# EUROPEAN PATENT APPLICATION

(11) **EP 2 562 212 A1**
(43) Date of publication of application: **27.02.2013**
(21) Application number: 11772131.6
(22) Date of filing: 21.04.2011
(51) Int. Cl.: C08L 1/08, A61K 8/02, A61K 8/20, A61K 8/24, A61K 8/73, A61K 47/36, A61L 31/00, C08K 3/00

(54) **HYDROGEL**

(30) Priority: 22.04.2010 JP 2010098942
(71) Applicant: Teijin Limited, Osaka-shi, Osaka 541-0054 (JP)
(72) Inventor: YAMAMOTO, Yuka, Hino-shi, Tokyo 191-0065 (JP); KANEKO, Hiroaki, Hino-shi, Tokyo 191-0065 (JP); ITO, Masaya, Hino-shi, Tokyo 191-0065 (JP); TANAKA, Taishi, Hino-shi, Tokyo 191-0065 (JP)
(74) Representative: Hallybone, Huw George
(86) International application number: PCT/JP2011/060342
(87) International publication number: WO 2011/132800

(57) **Abstract**

A hydrogel composed of a polyanionic polysaccharide derivative having a hydrophobic group introduced by covalent bonding and a salt solution with a salt concentration of 50 mM or more and 200 mM or less, the hydrogel having an aggregate structure with an average particle diameter of 100 to 2000 nm in the diluted solution, as well as a method for manufacturing the hydrogel comprising the steps of preparing a mixture containing a polyanionic polysaccharide derivative having a hydrophobic group introduced and a salt solution with a salt concentration of 50 mM or more and 200 mM or less and subjecting the mixture to heat treatment. The hydrogel of the present invention has a long-term stability and may be filtered using a porous filter having a pore diameter of 5 µm or more.

## Description

### Field of the Invention

The present invention relates to a hydrogel composed of polysaccharide derivatives having a long-term stability by virtue of formation of a specific aggregate structure and a manufacturing method thereof.

### Background Art

Chemically modified polysaccharides are used widely for foods, daily use articles, cosmetics, etc., as well as in medical field, by virtue of their hydrogel forming property, biocompatibility, and so on. Among them, hydrogel composed of polysaccharide derivatives is expected to be widely applied in the medical field, such as for wound dressing to keep the wound site in wet state, filling material for tissue restoration, post-surgical adhesion barrier, medium for cell culture, carrier for drug delivery system, etc.

Among the hydrogel for medical use, injectable gel having fluidity is desired as the safe and easy-to-handle hydrogel. Injectable gel having fluidity may be injected into a body through a syringe and the like and has a potential to be a medical material for less invasive endoscopic surgery.

These hydrogels should have a constant quality for a long term during transportation and storage after manufacturing. However, there is no known method to maintain the stability for some gel composed of polysaccharide derivatives known so far.

In the case that the hydrogel is injected through a syringe, it is desirable that the similar quality to the injectable solution for medical use be secured. For the injectable solution for medical use, the number of insoluble particles of a diameter of 10 µm or more is specified by US Pharmacopeia, European Pharmacopeia and Japanese Pharmacopeia. Also from a viewpoint of safety, it is required that the particles of a diameter of 10 µm or more be removed by filtration. However, it was difficult to remove insoluble particles from the injectable gel composed of polysaccharide derivatives known so far, because its solubility to water is low or its viscosity is high.

Japanese Patent Application Laid-Open Publication No. 2006-296916 describes an antiadhesive material composed of a product of a reaction between hyaluronic acid and phosphatidyl ethanolamine. International Publication WO2007/015579 describes an adhesion barrier composed of a product of a reaction between carboxymethylcellulose and phosphatidyl ethanolamine. International Publication WO1996/03751 describes an adhesion barrier composed of a hyaluronic acid derivative. International Publication WO2010/016611 describes a highly viscoelastic hydrogel obtained by dissolving the product of the reaction between carboxymethylcellulose and phosphatidyl ethanolamine to physiological saline.

However, long-term storage stability is not considered in any of the documents and there is no description or suggestion regarding the gel with high storage stability. In addition, there is no description or suggestion regarding the presence of foreign matters to be referred to later and the method to reduce them.

USP 6869938 describes a technique to filter a solution containing carboxymethylcellulose and polyethylene oxide using a filter during the process to prepare an aqueous adhesion barrier, although this is not a hydrogel. However, the filter used in this specification has a pore diameter of 30 µm or more, which is much larger than the filter to be mentioned later. Therefore, foreign matters with a diameter of several µm cannot be removed.

Japanese Patent Application Laid-Open Publication No. 2004-18750 describes that an aqueous solution of a hyaluronic acid derivative prepared by bonding "a nucleophilic reagent which may bond to carboxylic group" to hyaluronic acid was able to pass a porous filter (pore diameter 0.45 µm) by an alkaline treatment of the reaction solution, although it was not able to pass the filter before the alkaline treatment. However, this technique is not applicable to unstable compounds because this technique is performed under an alkaline condition. In addition, the alkaline solution should be neutralized in order to obtain an aqueous solution with a neutral pH. Furthermore, the specification neither describes nor suggests the enhancement of filterability by the heat treatment disclosed in the present specification.

### Summary of the Invention

The problem to be solved by the present invention is to provide a hydrogel composed of polysaccharide derivatives having a long-term stability.

As a result of the extensive investigation for the above-mentioned purpose, the present inventors have found that a hydrogel having a specific aggregate structure has a long-term stability and a good filterability and completed the present invention.

Thus, the present invention provides a hydrogel composed of a polyanionic polysaccharide derivative having a hydrophobic group introduced by covalent bonding and a salt solution with a salt concentration of 50 mM or more and 200 mM or less, the hydrogel having an aggregate structure with an average particle diameter of 100 to 2000 nm in the diluted solution.

Furthermore, the present invention provides a method for manufacturing the hydrogel, specifically a method for manufacturing the hydrogel comprising the steps of preparing a mixture containing a polyanionic polysaccharide derivative having a hydrophobic group introduced and a salt solution having a salt concentration of 50 mM or more and 200 mM or less and subjecting the mixture to heat treatment.

The hydrogel of the present invention has a long-term stability. Therefore, it has become possible to provide an article having a quality stable for a long term.

Furthermore, the hydrogel of the present invention may be filtered using a filter. Therefore, it has become possible to provide a hydrogel having a high safety by virtue of removal of foreign matters.

Furthermore, the hydrogel of the present invention is characterized in that it can be diluted in a solution state while maintaining the aggregate structure, because the hydrogel is formed by aggregation based on hydrophobic interaction, whereas the conventional hydrogel such as acrylamide gel, agarose, gelatin, etc. has a characteristic that the shape does not change unless temperature or pH is changed. Therefore, the aggregate structure of the hydrogel of the present invention may be determined by DLS measurement of the particle diameter of the aggregate in the diluted solution. As a result, it has been found that the long-term stability and filterability depend on the particle diameter of the aggregate.

In the present invention, a phrase "having an aggregate structure with an average particle diameter of 100 to 2000 nm in the diluted solution" means that the particle diameter of the aggregate in the diluted solution measured by DLS is 100 to 2000 nm.

Hereinafter, the structural difference between the conventional hydrogel and the hydrogel of the present invention will be discussed using schematic diagrams.

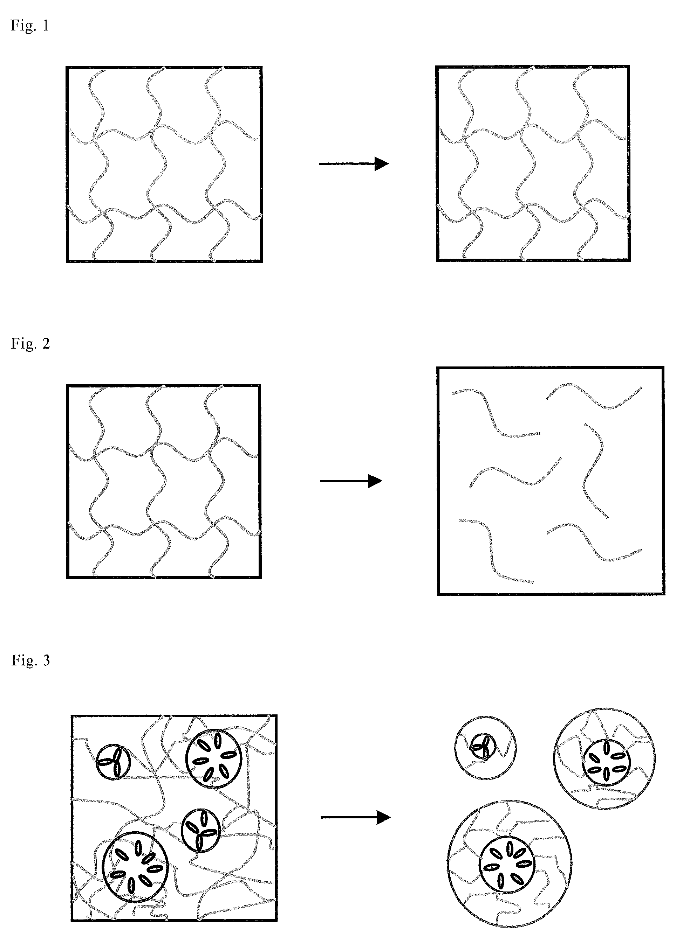

The left-hand side of Fig. 1 is a schematic diagram of the conventional hydrogel. The right-hand side of Fig. 1 shows a state after dilution for the purpose of analysis. The network structure does not change by dilution due to the hydrogel's property that the shape does not change unless temperature or pH changes. The hydrogel cannot be filtered at all. The left-hand side of Fig. 2 is a schematic diagram of a gel-like solution formed by physical entanglement of the molecular chain. The right-hand side of Fig. 2 shows a state after dilution for the purpose of analysis. The network structure has been disintegrated by dilution and the gel has become a solution state without formation of aggregate. Such gel-like solution has a problem of long-term stability.

The left-hand side of Fig. 3 is a schematic diagram of the hydrogel similar to the hydrogel of the present invention in that it has been formed by aggregation based on hydrophobic interaction, although the aggregate structure is not homogeneous. Such aggregate structure may be determined by DLS measurement of the particle diameter of the aggregate in the diluted solution. The right-hand side of Fig. 3 shows a state after dilution for the purpose of analysis. Particle diameter of the aggregate in the diluted solution is not homogeneous.

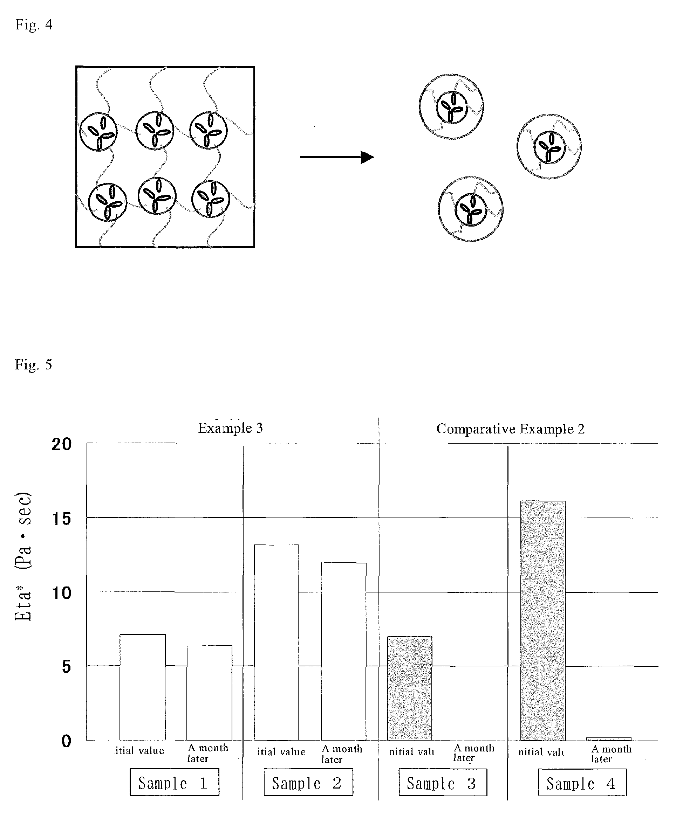

The left-hand side of Fig. 4 is a schematic diagram of the hydrogel of the present invention. The aggregate structure of such hydrogel is homogeneous and stable, thereby providing the excellent filterability and long-term stability. The right-hand side of Fig. 4 shows a state after dilution for the purpose of analysis of the aggregate structure. Particle diameter of the aggregate is homogeneous.

### Brief Description of the Drawings

Fig. 1 is a schematic diagram showing the structure of the conventional hydrogel obtained by crosslinking based on chemical bond.
Fig. 2 is a schematic diagram showing the structure of the conventional hydrogel formed by physical entanglement of the molecular chain.
Fig. 3 is a schematic diagram showing the structure of the conventional hydrogel formed by aggregation based on hydrophobic interaction.
Fig. 4 is a schematic diagram showing the structure of the hydrogel of the present invention having a homogeneous aggregate structure formed by hydrophobic interaction.
Fig. 5 shows the viscosity change (angular velocity 10 rad/sec) after the storage for one month at 40°C in Example 3 and Comparative Example 2.
Fig. 6 shows the result of viscoelasticity measurement before and after filtration in Example 4.
Fig. 7 shows the result of viscoelasticity measurement before and after filtration in Comparative Example 3.
Fig. 8 shows the change of the number of insoluble particles (per 1 ml of sample) before and after filtration in Example 7.
Fig. 9 shows the result of DLS measurement in Reference Example 1.

### Best Mode for Carrying Out the Invention

### [Polysaccharide derivatives]

In the hydrogel of the present invention and the manufacturing method thereof, polysaccharides used as the raw material for the polysaccharide derivatives are not particularly limited so long as they are polyanionic polysaccharide. Examples include natural polysaccharides such as hyaluronic acid, alginic acid, pectin, polygalacturonic acid, etc.; carboxyalkyl polysaccharides such as carboxymethylpullulan, carboxymethylchitin, carboxymethylchitosan, carboxymethylmannan, carboxymethylstarch, carboxymethyldextran, carboxyethylcellulose, carboxymethylcellulose, etc.; oxidized polysaccharides such as oxidized cellulose, oxidized starch, etc.; and sulfate group-containing polysaccharides such as chondroitin sulfate, dermatan sulfate, heparin, heparan sulfate, etc. Among them, carboxymethylcellulose and hyaluronic acid are preferable, carboxymethylcellulose being especially preferable.

Although weight average molecular weight of the raw material polysaccharides is not particularly limited, it is preferably 50,000 Da or more and 1,000,000 Da or less, more preferably 50,000 Da or more and 500,000 Da or less.

Although cations forming a salt with these polyanionic polysaccharides are not particularly limited, proton and metal ions, specifically metal ions such as sodium, potassium, lithium, calcium, magnesium, etc. and organic cations such as organic ammonium are exemplified. Among them, sodium salt of carboxymethylcellulose is preferable by virtue of its intended effect.

Although the hydrophobic group to be introduced to the polyanionic polysaccharides is not particularly limited, one having a phosphate ester structure is preferable, one having a phosphatidyl group represented by the following formula (I) being more preferable.

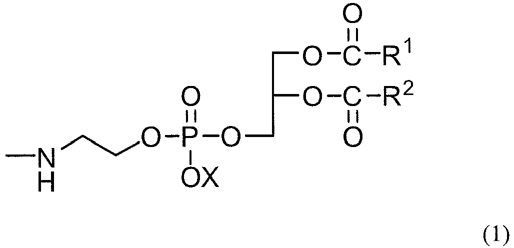

In formula (1), X is hydrogen atom or alkaline metal. R¹ and R² are each independently a linear or branched chain alkyl group or alkenyl group having 9 to 21 carbon atoms.

An example of the preferable polysaccharide derivatives is one having the repeat unit of the following formula (2).

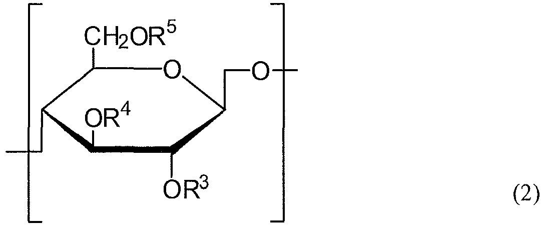

In formula (2), R³, R⁴ and R⁵ are each independently selected from the group consisting of the following formula (1-a), (1-b) and (1-c).

-H (1-a)

-CH₂COOX (1 - b)

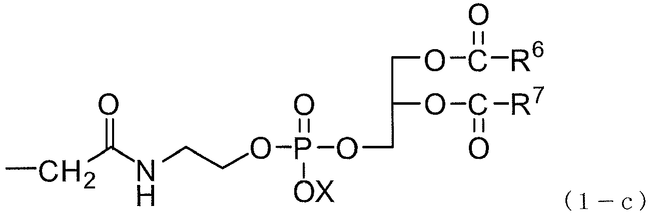

In formula (1-b) and formula (1-c), X is hydrogen atom or alkaline metal. In formula (1-c), R⁶ and R⁷ are each independently a linear or branched chain alkyl group or alkenyl group having 9 to 21 carbon atoms.

Although the equivalency of the substituent represented by formula (1-a) and the equivalency of the substituent represented by formula (1-b) are not particularly limited, it is preferable that the equivalency allows the polyanionic polysaccharide derivatives to dissolve in a salt solution containing 50 mM or more and 200 mM or less of sodium chloride. The equivalency of the substituent represented by formula (1-c) is preferably 0.25 to 5.0 mol%/sugar residue.

X in formula (1-b) and formula (1-c) is hydrogen atom or alkaline metal, preferably alkaline metal from a viewpoint of solubility. More preferably, X is sodium considering the safety in the living body.

R⁶ and R⁷ in formula (1-c) are each independently a linear or branched chain alkyl group or alkenyl group having 9 to 21 carbon atoms, preferably an alkenyl group having 9 to 21 carbon atoms from a viewpoint of solubility, more preferably an alkenyl group having 17 carbon atoms.

### [Hydrogel from polysaccharide derivatives]

The above-mentioned polysaccharide derivatives may be used to form a hydrogel by mixing them with a salt solution under a specific condition followed by heating (Hereinafter, such hydrogel may be briefly denoted as "hydrogel").

The salt solution to be used has a salt concentration of 50 mM or more and 200 mM or less, preferably is a salt solution containing 50 mM or more and 200 mM or less of sodium chloride, more preferably is a buffer solution containing 120 mM or more and 180 mM or less of sodium chloride with a pH adjusted to 6.5 or more and 7.5 or less, most preferably is a phosphate-buffered physiological saline. Although the solubility of macromolecules decreases with increase of salt strength due to the salting-out effect, it is possible to obtain the necessary strength by dissolving the polysaccharide derivatives to a phosphate-buffered physiological saline using the method of the present invention.

As for the concentration of the polysaccharide derivatives, hydrogel having appropriate viscoelasticity may be obtained with the polysaccharide derivatives used in the present invention in the amount of 0.1 to 5.0 weight parts, preferably 0.3 to 3.0 weight parts relative to 100 weight parts of water.

While usually any hydrogel may be obtained with the desired gel strength by increasing the polymer concentration, the hydrogel of the present invention may be obtained with a sufficient gel viscoelasticity even at a polymer concentration as low as 5 wt% or less relative to water. Specifically preferable physical properties of the hydrogel are represented by the viscoelasticity such that the hydrogel does not flow down when the vessel containing the hydrogel is tilted. Such hydrogel readily deforms when touched with a spatula and the like and may be readily applied to the affected area. Such hydrogel may also be injected using a device having a cannula such as a syringe.

As for the preferable viscoelasticity of the hydrogel, absolute viscosity when measured with a dynamic viscoelasticity measurement apparatus called rheometer under conditions of temperature of 37°C and angular velocity of 10 rad/sec is 0.5 to 100 Pa·sec, more preferably 2 to 30 Pa·sec. Although this range is suitable to simultaneously satisfy the handling property as the injectable gel and the retaining property in the body, this range may be changed as needed depending on the purpose of usage.

As for the aggregate particle diameter of the hydrogel of the present invention, the average particle diameter measured using a dynamic light scattering photometer under conditions of temperature of 20°C, measurement wavelength of 532.0 nm, concentration of the polysaccharide derivative of 0.3 wt%, and detection angle of 90.0° is preferably 100 to 2000 nm, more preferably 120 to 1500 nm, even more preferably 150 to 1200 nm, most preferably 200 to 1000 nm. This range is suitable to satisfy the handling property as the injectable gel and the retaining property in the body, as well as the long-term stability and filterability.

Heating temperature for manufacturing the hydrogel of the present invention is 70°C or higher and 140°C or lower, preferably 100°C or higher and 135°C or lower.

Although the heating time for manufacturing the hydrogel of the present invention is not particularly limited so long as it is longer than the shortest time required for the hydrogel to become filterable depending on the heating temperature, it is preferably 1 minute or longer and 3 hours or shorter, considering the thermal decomposition of the polysaccharide derivatives. Although the particle diameter distribution of the aggregate of such hydrogel shifts toward the smaller direction as the heat treatment proceeds, it will converge in a certain range over time.

Although the pressure during heating for manufacturing the hydrogel of the present invention is not particularly limited, so long as water does not reach the boiling point depending on the heating temperature, it is preferably atmospheric pressure to 10 atm.

The hydrogel of the present invention is filterable. More specifically, the hydrogel may be filtered with a porous filter having a pore diameter of 5 µm or more. Presumably this is because the average particle diameter of the aggregate of the polysaccharide derivative molecules in the hydrogel becomes smaller by heat treatment.

On the other hand, it has been confirmed that the average molecular weight of the polysaccharide derivatives in the hydrogel of the present invention did not decrease even by heat treatment.

Although the manufacturing method of the present invention is a method for manufacturing a hydrogel comprising the steps of preparing a mixture containing a polyanionic polysaccharide derivative having a hydrophobic group introduced and a salt solution with a salt concentration of 50 mM or more and 200 mM or less and subjecting this mixture to heat treatment, it is preferable that the manufacturing method of the present invention further comprise a step of filtering the mixture with a porous filter having a pore diameter of 5 µm or more after the heat treatment.

The hydrogel of the present invention may be applied to an adhesion barrier, medical use other than the adhesion barrier (wound dressing, filling material, medium for cell culture, carrier for drug delivery system, etc.), daily use products such as hair care products and moisturizing agent for skin, cosmetic use, and so on.

### Examples

(1) The materials used for the Examples and Comparative Examples are as follows.
   (i) Sodium carboxymethylcellulose (CMCNa: made by Dai-ichi Kogyo Seiyaku Co., Ltd.); P603A (Degree of substitution of hydroxyl group to carboxymethyl group 0.7, weight average molecular weight 250,000 Da), PRS (Degree of substitution of hydroxyl group to carboxymethyl group 0.8, weight average molecular weight 230,000 Da), PM250-L (Degree of substitution of hydroxyl group to carboxymethyl group 0.7, viscosity average molecular weight 540,000 Da)
   (ii) Tetrahydrofuran (THF: made by Wako Pure Chemical Industries, Ltd.)
   (iii) L-α-dioleoylphophatidylethanolamine (DOPE: made by NOF Corporation)
   (iv) 1-ethyl-3-(3-dimethylaminopropyl)carbodiimide hydrochloride (WSC·HCl: made by Osaka Synthetic Chemical Laboratories, Inc.)
   (v) 3,4-dihydro-3-hydroxy-4-oxo-1,2,3-benzotriazine (DHBT: made by Wako Pure Chemical Industries, Ltd.)
   (vi) N-hydroxysuccinimide (HOSu: made by Wako Pure Chemical Industries, Ltd.)
   (vii) Phosphate-buffered physiological saline;
      Sodium chloride (NaCl: made by Wako Pure Chemical Industries, Ltd.) 136.9 mM, Sodium dihydrogenphosphate (NaH₂PO₄·2H₂O: made by Wako Pure Chemical Industries, Ltd.) 2.92 mM, Sodium hydrogenphosphate hydrate (Na₂HPO₄·12H₂O: made by Wako Pure Chemical Industries, Ltd.) 9.02 mM
   (viii) Phosphate buffer;
      Sodium dihydrogenphosphate 2.92 mM, Sodium hydrogenphosphate hydrate 9.02 mM
   (ix) HEPES buffer;
      2-[4-(2-hydroxyethyl)-1-piperazinyl]ethanesulfonic acid (HEPES: made by GIBCO) 50 mM
(2) Measurement of phosphatidylethanolamine content in polysaccharide derivatives
   Content of phosphatidylethanolamine in polysaccharide derivatives was calculated from integral ratio of the peaks of ¹H-NMR spectra.
(3) Long-term stability test of the hydrogel
   Accelerated test was performed at 40°C. Viscosity of the hydrogel was measured just after preparation and after storage at 40°C using a viscoelasticity measurement apparatus RFSIII (made by TA Instruments, Japan).
(4) Measurement of viscosity before and after filtration
   The hydrogel filled in a syringe with a PVDF filter (pore diameter 5 µm) attached at the end was filtered by manual pushing at 25°C. Viscosity was measured using a viscoelasticity measurement apparatus RFSIII (made by TA Instruments, Japan) before and after filtration.
(5) Measurement of particle diameter by dynamic light scattering method
   Particle diameter distribution was measured using a fiber optical dynamic light scattering photometer FDLS-3000 (made by Otsuka Electronics Co., Ltd.) under the following conditions.
   Measurement conditions: Measurement temperature 20°C, Measurement wavelength 532.0 nm, Measurement concentration 0.3 wt%, Detection angle 90.0°, Number of integration cycles 100, Pretreatment: filtered with a PVDF filter (pore diameter 5 µm)
(6) Measurement of sugar concentration before and after filtration
   The hydrogel filled in a syringe with a PVDF filter (pore diameter 5µm) attached at the end was filtered by manual pushing at 25°C. Sugar concentration was measured by sulfuric acid anthrone method before and after filtration.
(7) Measurement of the number of insoluble particles before and after filtration
   The hydrogel filled in a syringe with a PVDF filter (pore diameter 5µm) attached at the end was filtered by manual pushing at 25°C. The number of insoluble particles was measured using an automatic particle counter for liquid System 9703-150 (made by HIAC) before and after filtration.
(8) Measurement of molecular weight of polysaccharide derivatives
   CMCNa was dissolved in 100 mM sodium chloride aqueous solution at a concentration of 0.02 to 0.05 wt% and its intrinsic viscosity was measured using an Ubbelohde viscometer to calculate the viscosity average molecular weight.

### [Example 1]

(1) Synthesis of raw material
   To a mixed solvent of water and tetrahydrofuran 1:1 (volume ratio), 3000 mg of CMCNa (PRS) was dissolved. To this solution was added 349 mg (0.50 mmol) of DOPE, 143 mg (0.74 mmol) of WSC·HCl, and 324 mg (1.98 mmol) of DHBT. After the reaction was completed, the polysaccharide derivative was obtained by filtering out a precipitate formed by adding the reaction mixture to ethanol. The solid obtained was washed with ethanol and dried under reduced pressure. Degree of substitution of the polysaccharide derivative obtained with DOPE was 1.47 mol%/sugar residue.
(2) Preparation of hydrogel
   To 1980 mg of phosphate-buffered physiological saline, 20 mg of the polysaccharide derivative synthesized in (1) was dissolved to prepare a solution at a concentration of 1.0 wt%. The mixture was heated for 20 minutes at 121°C.
(3) Measurement of viscosity and average particle diameter of the hydrogel
   Viscosity of the sample prepared in (2) was measured at an angular velocity of 10 rad/sec. Absolute viscosity was about 7.1 Pa·sec, showing that a hydrogel with high viscosity was prepared. This hydrogel is referred to as Sample 1. Average particle diameter of a diluted solution of this sample was measured to be 580 nm.

### [Example 2]

(1) Synthesis of raw material
   To a mixed solvent of water and tetrahydrofuran 1:1 (volume ratio), 3000 mg of CMCNa (PM250-L) was dissolved. To this solution was added 384 mg (0.52 mmol) of DOPE, 99 mg (0.52 mmol) of WSC·HCl, and 337 mg (2.06 mmol) of DHBT. After the reaction was completed, the polysaccharide derivative was obtained by filtering out a precipitate formed by adding the reaction mixture to ethanol. The solid obtained was washed with ethanol and dried under reduced pressure. Degree of substitution of the polysaccharide derivative obtained with DOPE was 0.76 mol%/sugar residue.
(2) Preparation of hydrogel
   To 1980 mg of phosphate-buffered physiological saline, 20 mg of the polysaccharide derivatives synthesized in (1) was dissolved to prepare a solution at a concentration of 1.0 wt%. The mixture was heated for 20 minutes at 121°C.
(3) Measurement of viscosity and average particle diameter
   Viscosity of the sample prepared in (2) was measured at an angular velocity of 10 rad/sec. Absolute viscosity was about 13.1 Pa·sec, showing that a hydrogel with high viscosity was prepared. This hydrogel is referred to as Sample 2. Average particle diameter of a diluted solution of this sample was measured to be 1789 nm.

### [Comparative Example 1]

(1) Raw material
   The polysaccharide derivatives synthesized in Example 1 and Example 2 were used.
(2) Preparation of hydrogel (prepared using water as a solvent)
   To 1980 mg of water, 20 mg each of the polysaccharide derivatives synthesized in Example 1 and Example 2 was dissolved to prepare a solution at a concentration of 1.0 wt%. The mixture was heated for 20 minutes at 121°C.
(3) Measurement of viscosity and average particle diameter
   Viscosity of the samples prepared was measured at an angular velocity of 10 rad/sec. Absolute viscosity was 0.1 Pa·sec or less for the sample made using the polysaccharide derivative synthesized in Example 1 and about 0.3 Pa·sec for the sample made using the polysaccharide derivative synthesized in Example 2, showing that neither samples were gelled. Measurement of average particle diameter for the diluted solutions of these samples showed that the scattering intensity was insufficient and that the aggregate was not formed.

### [Example 3]

(1) Raw material
   The polysaccharide derivatives synthesized in Example 1 and Example 2 were used.
(2) Preparation of hydrogel
   To 1980 mg of phosphate-buffered physiological saline, 20 mg each of the polysaccharide derivatives synthesized in Example 1 and Example 2 was dissolved to prepare a solution at a concentration of 1.0 wt%. The mixture was heated for 20 minutes at 121°C.
(3) Long-term stability test
   Viscosity of the samples prepared in (2) was measured just after preparation and after storage at 40°C for one month. Absolute viscosity of both samples measured at an angular velocity of 10 rad/sec was not changed. Results are shown in Fig. 5.

### [Comparative Example 2]

(1) Raw material
   To a mixed solvent of water and tetrahydrofuran 1:1 (volume ratio), 3000 mg of CMCNa (PM250-L) was dissolved. To this solution was added 351 mg (0.47 mmol) of DOPE, 399 mg (2.08 mmol) of WSC·HCl, and 239 mg (2.08 mmol) of HOSu. After the reaction was completed, the polysaccharide derivative was obtained by filtering out a precipitate formed by adding the reaction mixture to ethanol. The solid obtained was washed with ethanol and dried under reduced pressure. Degree of substitution of the polysaccharide derivative obtained with DOPE was 2.39 mol%/sugar residue. In addition, a polysaccharide derivative having a degree of substitution with DOPE of 2.79 mol%/sugar residue was similarly synthesized.
(2) Preparation of hydrogel (prepared using water as a solvent)
   To 1980 mg of water, 20 mg of the polysaccharide derivatives synthesized in (1) was dissolved to prepares solution at a concentration of 1.0 wt%. The mixture was heated for 20 minutes at 121°C.
(3) Long-term stability test and measurement of average particle diameter
   Viscoelasticity of the samples prepared in (2) was measured just after preparation and after storage at 40°C for one month. Absolute viscosity measured at an angular velocity of 10 rad/sec was significantly changed. The results with Sample 3, which was a hydrogel prepared using the polysaccharide derivative having a degree of substitution with DOPE of 2.39 mol%/sugar residue, and Sample 4, which was a hydrogel prepared using the polysaccharide derivative having a degree of substitution with DOPE of 2.79 mol%/sugar residue, are shown in Fig. 5. Measurement of average particle diameter for the diluted solutions of these samples just after preparation showed that the scattering intensity was insufficient and that the aggregate was not formed.

### [Example 4]

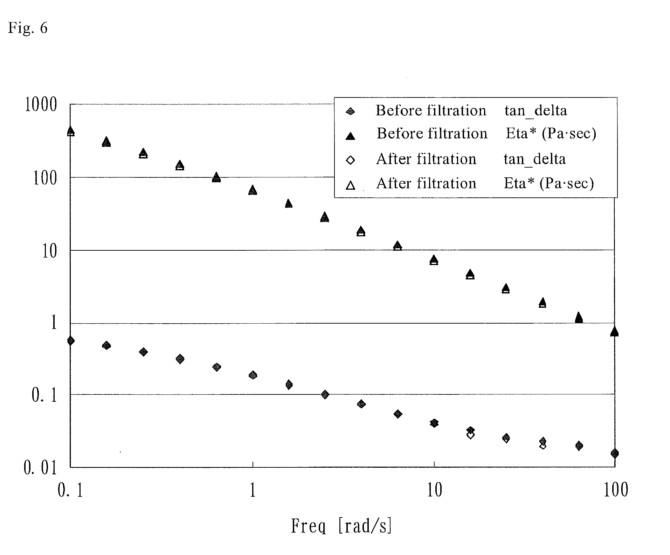

(1) Synthesis of raw material
   To a mixed solvent of water and tetrahydrofuran 1:1 (volume ratio), 3000 mg of CMCNa (P603A) was dissolved. To this solution was added 384 mg (0.52 mmol) of DOPE, 247 mg (1.29 mmol) of WSC·HCl, and 337 mg (2.06 mmol) of DHBT. After the reaction was completed, the polysaccharide derivative was obtained by filtering out a precipitate formed by adding the reaction mixture to ethanol. The solid obtained was washed with ethanol and dried under reduced pressure. Degree of substitution of the polysaccharide derivative obtained with DOPE was 1.30 mol%/sugar residue.
(2) Preparation of hydrogel
   To 1980 mg of phosphate-buffered physiological saline, 20 mg of the polysaccharide derivative synthesized in (1) was dissolved to prepare a solution at a concentration of 1.0 wt%. The mixture was heated for 5 minutes at 121°C.
(3) Measurement of viscosity before and after filtration and average particle diameter
   Viscoelasticity of the sample prepared in (2) was measured before and after filtration. There was no change in viscoelasticity after filtration. Results are shown in Fig. 6 and Table 1. Average particle diameter of the diluted solution of this sample was measured to be 446 nm.

### [Comparative Example 3]

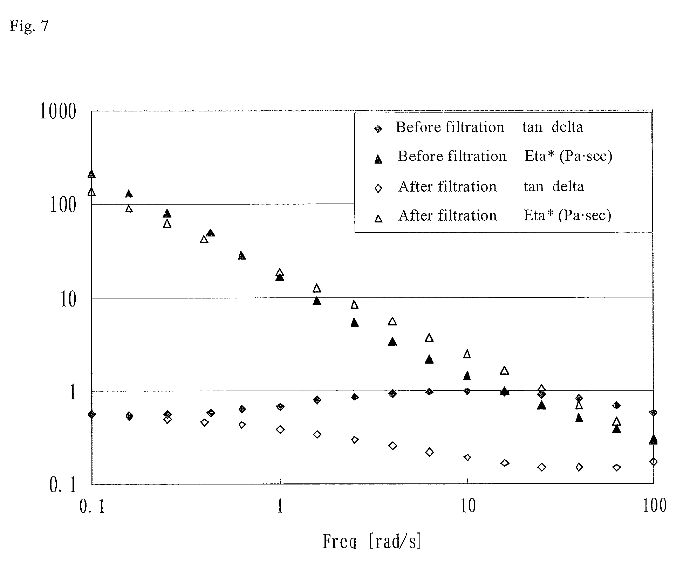

(1) Raw material
The polysaccharide derivative synthesized in Example 4 was used.
(2) Preparation of hydrogel (prepared without heat treatment)
To 1980 mg of phosphate-buffered saline, 20 mg of the above-mentioned polysaccharide derivative was dissolved to prepare a hydrogel at a concentration of 1.0 wt%.
(3) Measurement of viscosity before and after filtration and average particle diameter
Viscoelasticity of the sample prepared in (2) was measured before and after filtration. There was a significant change in viscoelasticity before and after filtration. Results are shown in Fig. 7 and Table 1.
Average particle diameter of the diluted solution of this sample was measured to be 2820 nm.

**[Table 1]**

| Change in viscoelasticity before and after filtration in Example 4 and Comparative Example 3 (Angular velocity 10 rad/sec) | | | | |
|---|---|---|---|---|
| | Example 4 | | Comparative Example 3 | |
| | Eta* [Pa·sec] | tan Δ | Eta* [Pa·sec] | tan Δ |
| Before filtration | 7.63 | 0.04 | 1.45 | 0.98 |
| After filtration | 7.20 | 0.04 | 2.49 | 0.19 |

### [Example 5]

(1) Raw material
   CMCNa (P603A) was used.
(2) Preparation of hydrogel diluted solution
   To 1980 mg of phosphate-buffered physiological saline, 20 mg of CMCNa (P603A) was dissolved to prepare a solution at a concentration of 1.0 wt%. This solution was divided into two aliquots, one with a heat treatment at 121°C for 20 minutes and one without the heat treatment. Each solution was diluted with 100 mM sodium chloride aqueous solution to prepare solutions at a concentration of 0.05 to 0.02 wt%.
(3) Measurement of molecular weight of polysaccharide derivatives
   Viscosity average molecular weight was measured from intrinsic viscosity for the samples with and without heat treatment. The viscosity average molecular weight of the sample with heat treatment and the sample without heat treatment was 105,000 Da and 115,000Da, respectively. Since the viscosity average molecular weight of these samples is similar, it was confirmed that the molecular weight of the polysaccharide derivatives did not change with heat treatment.

### [Example 6]

(1) Synthesis of raw material
To a mixed solvent of water and tetrahydrofuran 1:1 (volume ratio), 3000 mg of CMCNa (PRS) was dissolved. To this solution was added 349 mg (0.47 mmol) of DOPE, 99 mg (0.52 mmol) of WSC·HCl, and 337 mg (2.06 mmol) of DHBT. After the reaction was completed, the polysaccharide derivative was obtained by filtering out a precipitate formed by adding the reaction mixture to ethanol. The solid obtained was washed with ethanol and dried under reduced pressure. Degree of substitution of the polysaccharide derivative obtained with DOPE was 1.57 mol%/sugar residue. Similarly, a polysaccharide derivative having a degree of substitution of 1.07 mol%/sugar was synthesized.
(2) Preparation of hydrogel
To 1980 mg of phosphate-buffered physiological saline, 20 mg of the polysaccharide derivatives synthesized in (1) was dissolved to prepare a solution at a concentration of 1.0 wt%. The mixture was heated for 5 minutes at 121°C and filtered with a PVDF filter (pore diameter 5µm).
(3) Measurement of sugar concentration before and after filtration and average particle diameter
Sugar concentration of the samples prepared in (2) was measured before and after filtration. There was no change in sugar concentration before and after filtration. Results are shown in Table 2. Average particle diameter of the diluted solution of these samples was 224 nm for the sample with degree of substitution of DOPE of 1.07 mol%/sugar residue and 648nm for the sample with degree of substitution of 1.57 mol%.

**[Table 2]**

| Change of sugar concentration before and after filtration in Example 6 | |
|---|---|
| PE Degree of substitution [mol%/sugar] | Sugar concentration after filtration/before filtration |
| 1.07 | 1.03 |
| 1.57 | 0.97 |

### [Example 7]

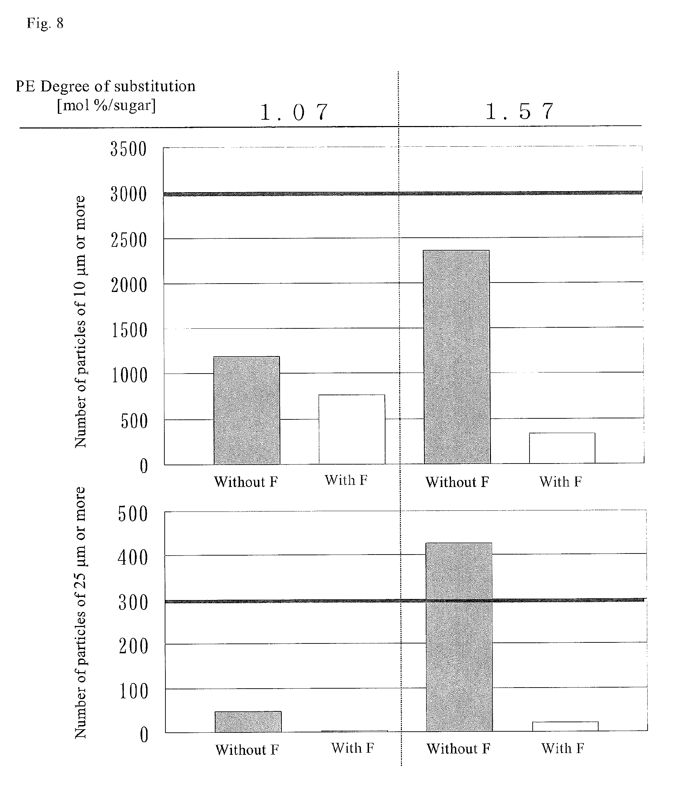

(1) Hydrogel
   The samples prepared in Example 6 were used.
(2) Measurement of the number of insoluble particles before and after filtration
   The number of insoluble particles of a diameter of 10 µm or more and 25 µm or more was measured for 1 mL of the samples from (1) before and after filtration. As a result, the number of insoluble particles of a diameter of 10 µm or more and 25 µm or of the samples decreased by one-half to one-twentieth compared to the control substance. Results are shown in Fig. 8.

### [Example 8]

(1) Synthesis of raw material
   To a mixed solvent of water and tetrahydrofuran 1:1 (volume ratio), 3000 mg of CMCNa (PRS) was dissolved. To this solution was added 349 mg (0.50 mmol) of DOPE, 143 mg (0.74 mmol) of WSC·HCl, and 324 mg (1.98 mmol) of DHBT. After the reaction was completed, the polysaccharide derivative was obtained by filtering out a precipitate formed by adding the reaction mixture to ethanol. The solid obtained was washed with ethanol and dried under reduced pressure. Degree of substitution of the polysaccharide derivative obtained with DOPE was 1.44 mol%/sugar residue.
(2) Preparation of hydrogel
   To 1980 mg of a phosphate buffer containing 200 mM sodium chloride, 20 mg of the polysaccharide derivative synthesized in (1) was dissolved to prepare a solution at a concentration of 1.0 wt%. The mixture was heated for 5 minutes at 121°C, filtered with a PVDF filter (pore diameter 5 µm), and heated again for 20 minutes at 121°C.
(3) Measurement of viscosity before and after filtration and average particle diameter
   Viscoelasticity of the sample prepared in (2) was measured before and after filtration. There was no change in absolute viscosity measured at an angular velocity of 10 rad/sec after filtration. Results are shown in Table 3. Average particle diameter of the diluted solution of this sample was measured to be 620 nm.
(4) Long-term stability test
   Viscoelasticity of the sample prepared in (2) was measured just after preparation and after storage at 40°C for one month. Absolute viscosity measured at an angular velocity of 10 rad/sec was not changed. Results are shown in Table 3.

### [Example 9]

(1) Raw material
The polysaccharide derivative synthesized in Example 8 was used.
(2) Preparation of hydrogel
To 1980 mg of a HEPES buffer containing 137 mM sodium chloride, 20 mg of the above-mentioned polysaccharide derivative was dissolved to prepare a solution at a concentration of 1.0 wt%. The mixture was heated for 5 minutes at 121°C, filtered with a PVDF filter (pore diameter 5 µm), and heated again for 20 minutes at 121°C.
(3) Measurement of viscosity before and after filtration and average particle diameter
Viscoelasticity of the sample prepared in (2) was measured before and after filtration. There was no change in absolute viscosity measured at an angular velocity of 10 rad/sec after filtration. Results are shown in Table 3. Average particle diameter of the diluted solution of this sample was measured to be 887 nm.
(4) Long-term stability test
Viscoelasticity of the sample prepared in (2) was measured just after preparation and after storage at 40°C for one month. Absolute viscosity measured at an angular velocity of 10 rad/sec was not changed. Results are shown in Table 3.

**[Table 3]**

| Change in viscoelasticity before and after filtration and after storage at 40°C for one month in Examples 8 and 9 and Comparative Examples 4 and 5 (angular velocity 10 rad/sec) | | | | | | |
|---|---|---|---|---|---|---|
| | Solvent | Heating condition | Eta* [Pa·sec] | | | Average particle diameter [nm] |
| | | | Just after gel preparation | | Storage at 40°C for one month | |
| | | | Before filtration | After filtration | | |
| Example 8 | Phosphate buffer containing 200 mM NaCl | 121°C, 5 min | 4.8 | 4.5 | 4.4 | 620 |
| Example 9 | HEPES buffer containing 137 mM NaCl | | 7.3 | 6.8 | 6.5 | 887 |
| Comparative Example 4 | Phosphate buffer | | 0.1 or less | - | - | No aggregate formed |
| Comparative Example 5 | HEPES buffer | | 0.2 | - | - | No aggregate formed |

### [Comparative Example 4]

(1) Raw material
   The polysaccharide derivative synthesized in Example 8 was used.
(2) Preparation of hydrogel (prepared using phosphate buffer as a solvent)
   To 1980 mg of phosphate buffer, 20 mg of the above-mentioned polysaccharide derivative was dissolved to prepare a solution at a concentration of 1.0 wt%. The mixture was heated for 5 minutes at 121°C, filtered with a PVDF filter (pore diameter 5 µm), and heated again for 20 minutes at 121°C.
(3) Measurement of viscosity and average particle diameter
   Viscoelasticity of the sample prepared in (2) was measured at an angular velocity of 10 rad/sec. Absolute viscosity was about 0.1 Pa·sec or less, showing that the sample was not gelled. Measurement of average particle diameter for the diluted solution of this sample showed that the scattering intensity was insufficient and that the aggregate was not formed.

### [Comparative Example 5]

(1) Raw material
   The polysaccharide derivative synthesized in Example 9 was used.
(2) Preparation of hydrogel (prepared using HEPES buffer as a solvent)
   To 1980 mg of HEPES buffer, 20 mg of the above-mentioned polysaccharide derivative was dissolved to prepare a solution at a concentration of 1.0 wt%. The mixture was heated for 5 minutes at 121°C, filtered with a PVDF filter (pore diameter 5 µm), and heated again for 20 minutes at 121°C.
(3) Measurement of viscosity and average particle diameter
   Viscoelasticity of the sample prepared in (2) was measured at an angular velocity of 10 rad/sec. Absolute viscosity was about 0.3 Pa·sec, showing that the sample was not gelled. Measurement of average particle diameter for the diluted solution of this sample showed that the scattering intensity was insufficient and that the aggregate was not formed.

### [Reference Example 1]

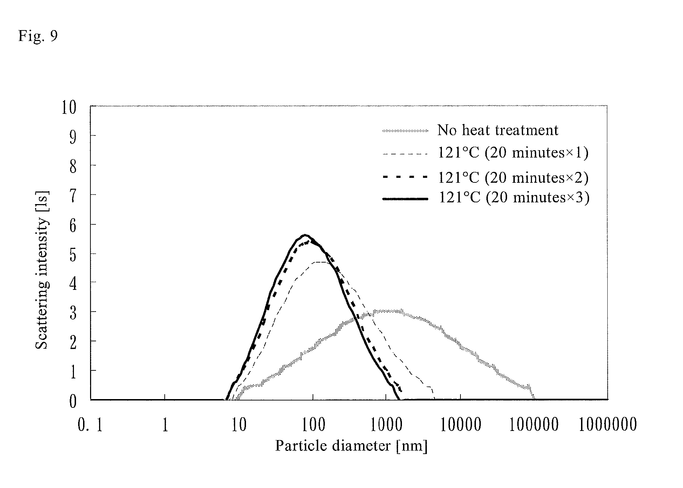

(1) Raw material
   The polysaccharide derivative synthesized in Example 4 was used.
(2) Preparation of hydrogel
   To 1980 mg of phosphate-buffered physiological saline, 20 mg of the above-mentioned polysaccharide derivative was dissolved to prepare a solution at a concentration of 1.0 wt%. The mixture was repeatedly heated for 20 minutes at 121°C three times.
(3) DLS Measurement
   DLS measurement was performed for the sample prepared in (2). Results are shown in Fig. 9. Although the particle diameter distribution shifted toward the smaller direction by a treatment at 121°C for 20 minutes, the change in the particle diameter distribution became less significant to converge in a certain range even after repeating the same treatment.

### Industrial Applicability

The hydrogel of the present invention may be used, for example, as medical articles such as antiadhesive material, a DDS carrier, etc.

## Claims

1. A hydrogel comprising a polyanionic polysaccharide derivative having a hydrophobic group introduced by covalent bonding and a salt solution having a salt concentration of 50 mM or more and 200 mM or less, the hydrogel having an aggregate structure with an average particle diameter of 100 to 2000 nm in the diluted solution.

2. The hydrogel according to claim 1, wherein the polyanionic polysaccharide is a polysaccharide having a carboxymethyl group.

3. The hydrogel according to claim 1, wherein the polyanionic polysaccharide is carboxymethylcellulose.

4. The hydrogel according to any of claims 1 to 3, wherein the hydrophobic group has a phosphate ester structure.

5. The hydrogel according to any of claims 1 to 3, wherein the hydrophobic group has a phosphatidyl group represented by the following formula (1): 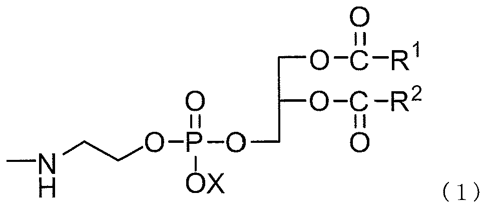 wherein X is hydrogen atom or alkaline metal; R¹ and R² are each independently a linear or branched chain alkyl group or alkenyl group having 9 to 21 carbon atoms.

6. The hydrogel according to any of claims 1 to 5, wherein the manufacturing process of the hydrogel comprises a heating step at 70°C or higher and 140°C or lower and the average particle diameter of the aggregate is 120 to 1500 nm.

7. The hydrogel according to any of claims 1 to 5, wherein the manufacturing process of the hydrogel comprises a heating step at 100°C or higher and under a pressurized condition at atmospheric pressure to 10 atm and the average particle diameter of the aggregate is 150 to 1200 nm.

8. The hydrogel according to any of claims 1 to 7, wherein the equivalency of the hydrophobic group is 0.25 to 5.0 mol%/sugar residue.

9. The hydrogel according to any of claims 1 to 8, wherein the solvent is a salt solution containing 50 mM or more and 200 mM or less of sodium chloride.

10. The hydrogel according to any of claims 1 to 8, wherein the solvent is a buffer containing 120 mM or more and 180 mM or less of sodium chloride with a pH adjusted to 6.5 or more and 7.5 or less and the average particle diameter of the aggregate is 200 to 1000 nm.

11. The hydrogel according to any of claims 1 to 8, wherein the solvent is a phosphate-buffered physiological saline.

12. The hydrogel according to any of claims 1 to 11, wherein the concentration of the polysaccharide derivative is 0.3 to 3.0 wt%.

13. A hydrogel obtained by filtrating the hydrogel according to any of claims 1 to 12 with a porous filter having a pore diameter of 5 µm or more.

14. The hydrogel according to claim 13, wherein the number of insoluble particles of a diameter of 10 µm or more is 3000 or less per 1 mL and/or the number of insoluble particles of a diameter of 25 µm or more is 300 or less per 1 mL.

15. A method for manufacturing a hydrogel, comprising the steps of preparing a mixture containing a polyanionic polysaccharide derivative having a hydrophobic group introduced and a salt solution with a salt concentration of 50 mM or more and 200 mM or less and subjecting this mixture to heat treatment.

16. The manufacturing method according to claim 15, wherein the step of the heat treatment is a step of treatment at 70 to 140°C and for 1 minute or longer and 3 hours or shorter.

17. The manufacturing method according to claim 15 or 16, wherein the salt solution is physiological saline.

18. The manufacturing method according to any of claims 15 to 17, comprising a step of further filtering the hydrogel with a porous filter having a pore diameter of 5 µm or more after the heat treatment.
